# FASCICULE DE BREVET EUROPEEN

(11) **EP 4 081 291 B1**
(45) Date de publication et mention de la délivrance du brevet: **07.02.2024**
(21) Numéro de dépôt: 20851220.2
(22) Date de dépôt: 22.12.2020
(51) Int. Cl.: A61N 5/10

(54) **DISPOSITIF D'IRRADIATION COLLABORATIF**
KOLLABORATIVE BESTRAHLUNGSVORRICHTUNG
COLLABORATIVE IRRADIATING DEVICE

(30) Priorité: 26.12.2019 FR 1915627
(43) Date de publication de la demande: 02.11.2022
(73) Titulaire: THERYQ, 13790 Peynier (FR)
(72) Inventeur: LIGER, Philippe, 13790 PEYNIER (FR); DELMAS, Marc, 13320 BOUC BEL AIR (FR); BRAU, Emmanuel, 04100 MANOSQUE (FR)
(74) Mandataire: Santarelli
(86) Numéro de dépôt international: PCT/FR2020/052603
(87) Numéro de publication internationale: WO 2021/130450

(56) Documents cités:
- WO-A1-2016/054256
- DE-A1-102019 115 744
- US-A1- 2012 035 470
- US-A1- 2016 183 899
- US-A1- 2019 314 645

## Description

La présente demande concerne un dispositif d'irradiation, notamment pour la radiothérapie, collaboratif.

Un tel dispositif est aussi appelé « cobot ».

La radiothérapie, externe, ou peropératoire (RTPO ou IORT : intraoperative radiotherapy), est une méthode de traitement locorégional des cancers. Elle est avec la chirurgie, le traitement le plus fréquent des cancers et peut entrainer une rémission nette à elle seule. Elle peut être utilisée seule ou associée à la chirurgie et à la chimiothérapie. Ses indications sont liées au type de tumeur, à sa localisation, à son stade et à l'état général de la cible (en général une zone à traiter d'un patient). Elle présente dans certains cas, l'avantage d'être réalisée en ambulatoire par le fait que les séances peuvent être de courte durée et les effets secondaires moindres que ceux d'une chimiothérapie.

Pour ce faire, la radiothérapie utilise une irradiation de rayonnement ionisant (rayon X, électrons, protons, etc). pour détruire les cellules cancéreuses en affectant leur capacité à se reproduire. L'irradiation a pour but de détruire toutes les cellules tumorales tout en épargnant les tissus sains périphériques. Toutefois, pour certains types de cancer, un traitement par rayons X présente la difficulté que la tumeur peut être localisée très près de parties d'organes qu'il est préférable d'éviter d'irradier.

En outre, il est apparu que le fait de délivrer de très fortes doses en des temps très courts (typiquement moins d'une seconde) est bien moins néfaste pour les tissus sains que de délivrer la même dose, voire une dose moindre, en un temps plus long (i.e. de plusieurs secondes, voire plusieurs minutes, pour un mode de traitement classique). Ce phénomène est décrit par exemple dans l'article « The Advantage of FLASH Radiotherapy Confirmed in Mini-pig and Cat-cancer Patients », Marie-Catherine Vozenin et al., Clin Cancer Res 2018, American Association for Cancer Research.

Un tel mode de traitement est notamment désigné « mode flash ».

Une radiothérapie en mode flash permet ainsi de produire les mêmes effets thérapeutiques qu'une radiothérapie classique tout en limitant des effets secondaires indésirables possibles.

En outre, la radiothérapie en mode flash peut permettre d'éviter d'avoir à retirer la tumeur au préalable, ce qui est particulièrement commode si la tumeur n'est pas opérable (par exemple lorsqu'une tumeur est située à proximité de la carotide ou du pancréas d'un patient car il y a alors trop de risques de toucher le système nerveux).

La radiothérapie en mode flash permet ainsi de traiter une plus grande variété de tumeurs, en particulier des tumeurs traditionnellement non opérables (par exemple par les moyens conventionnels, par exemple par un scalpel).

Il est aussi nécessaire de délivrer, mesurer et/ou contrôler précisément les fortes doses de rayonnement ionisant administrées en des durées très brèves. Les impacts d'un mauvais contrôle de dose et/ou du débit de dose absorbée par la cible peuvent conduire à une destruction de cellules, tissus ou organes sains et les effets secondaires qui en découlent peuvent avoir, dans certains cas, des conséquences néfastes sur des organes à risques.

Le document EP3071292 décrit par exemple un dispositif d'irradiation à rayonnement ionisant pour la radiothérapie et/ou la radiobiologie permettant de réaliser du mode flash. Le dispositif comprend alors un accélérateur linéaire de faisceau d'électrons ou d'ions, appelé couramment « LINAC », et une électronique de contrôle et de commande qui permet d'arrêter globalement l'émission de rayonnement ionisant lorsque la dose prescrite par l'opérateur est atteinte, et plus particulièrement un dispositif d'irradiation à rayonnement ionisant configuré pour délivrer, de manière contrôlée et précise, une dose de rayonnement ionisant d'au moins 0,25 Gy (gray), de préférence 10 Gy, dans une gamme d'énergie comprise entre 1 MeV et 50 MeV, en des instants très brefs, c'est-à-dire par exemple en moins de 100 ms, voire moins de 1 ms, voire moins de 100 µs, même encore moins de 0,1 µs. Il concerne également un dispositif d'irradiation à rayonnement ionisant doté d'un système de contrôle d'impulsion de puissance capable de produire un faisceau de particules d'énergie ajustable dans une gamme comprise entre 1 MeV et 50 MeV, pulsé à une fréquence (f) souhaitée avec une durée d'impulsion (d) ajustable, et capable de délivrer un débit de dose absorbée d'au moins 250 Gy/s, voire 500 Gy/s ou même encore d'au moins 1000 Gy/s dans un champ d'exposition de quelques cm² (centimètre-carré) à 10 cm².

En pratique, le faisceau émis par la source de rayonnement (par exemple le LINAC) doit être appliqué selon un angle et une distance particuliers par rapport à la cible.

Il existe des dispositifs de radiothérapie classique qui comportent par exemple un robot 6-axes dont une extrémité supporte un LINAC.

Généralement, un praticien positionne un applicateur sur la cible (sur le patient, ou en partie dans le patient selon le cas) et le maintient à la main. Puis, un autre praticien bouge le dispositif pour que la source de rayonnement en sortie du LINAC se raccorde à l'applicateur. Ensuite, chacun sort de la salle où se trouve le patient et le rayonnement est activé pour réaliser le traitement. Pour cela, une interface homme-machine (IHM) est généralement organisée dans une salle dite « salle de commande » qui est située à côté d'une salle dite « salle d'opération » dans laquelle se trouvent le patient et le dispositif d'irradiation. La salle d'opération est alors munie d'une enceinte de radioprotection.

Le document US2019/0314645 décrit par exemple un dispositif de radiothérapie comportant un robot 6-axes configuré pour aligner une tête de traitement avec un applicateur fixé à une table d'opération.

La procédure de manipulation du dispositif d'irradiation est donc très longue et délicate car le LINAC doit être orienté selon des prescriptions particulières par rapport à la cible (donc par rapport à l'applicateur) alors que le dispositif est très lourd et difficile à manipuler.

En conséquence, des dispositifs automatisés ont été développés, par exemple des dispositifs capables de détecter une cible, ou par exemple guidés par un système externe, ou bien télécommandés (par exemple via un joystick). Cependant, non seulement un tel dispositif est difficile à télécommander, mais de plus, il est généralement plus agréable et plus pratique pour un praticien de pouvoir manipuler directement le dispositif d'irradiation.

En outre, pour réaliser des traitements en mode flash, il est nécessaire de disposer de davantage de puissance qu'en radiothérapie classique, induisant d'autant plus de poids dans le dispositif d'irradiation, ce qui contrevient aux facilités de manipulation du dispositif.

Le but de la présente invention est ainsi de fournir un dispositif d'irradiation à rayonnement ionisant, notamment pour la radiothérapie et/ou radiobiologie, surmontant au moins en partie les inconvénients précités.

L'invention est définie dans les revendications attenantes.

A cet effet, est proposé, un dispositif d'irradiation, configuré pour irradier une cible, en mode flash ou de manière conventionnelle, comportant :
- Un robot 6-axes comportant une base et un bras 6-axes dont une première extrémité est fixée à la base et une seconde extrémité est dite extrémité libre ;
- Un système d'irradiation, comportant une source hyperfréquence et une source de rayonnement alimentée par la source hyperfréquence, le système d'irradiation étant positionné à l'extrémité libre du bras 6-axes ;
- Une poignée de manipulation, solidaire de la source de rayonnement, comportant un applicateur configuré pour être fixé en sortie de la source de rayonnement
- Au moins un capteur d'effort placé entre la poignée de manipulation et le bras 6-axes, voire entre la poignée de manipulation et la source de rayonnement ; et
- Une unité de contrôle-commande configurée pour recevoir des informations du capteur d'effort et commander le bras 6-axes en fonction des informations du capteur d'effort reçues.

L'au moins un capteur d'effort convertit l'effort auquel il est soumis en signal électrique.

La mesure de signal électrique émis par le capteur d'effort est convertie en commande de déplacement et d'orientation de la source de rayonnement. Un effort soumis à un capteur dans une direction X est converti en une commande de déplacement dans la direction X avec une accélération et une vitesse qui dépendent de l'intensité de l'effort, c'est-à-dire de l'amplitude du signal électrique émis par le capteur d'effort dans la direction X.

Lorsque deux capteurs d'effort positionnés sur un axe de direction Y sont soumis à des efforts d'intensités différentes selon une même direction X, alors la différence d'intensité correspond à un couple exercé selon un axe de direction Z perpendiculaire au plan qui contient l'axe de direction Y, sur lequel sont positionnés les capteurs d'effort, et la direction X.

Cette différence d'intensité d'efforts se traduit par une différence d'amplitude de signaux émis par les capteurs d'effort dans la direction X.

Cette différence d'amplitude de signaux électriques est alors convertie en une commande de rotation selon un axe de direction Z avec une accélération et une vitesse qui dépendent de la différence d'intensité des efforts, c'est-à-dire de la différence des amplitudes des signaux électriques émis par les deux capteurs d'effort dans la direction X.

Les capteurs d'effort permettent ainsi un asservissement du dispositif d'irradiation de sorte que le dispositif d'irradiation forme alors un robot collaboratif, ou « cobot », dont la manipulation est assistée.

Le dispositif peut par exemple être manipulé par une seule personne alors qu'au moins deux personnes étaient généralement nécessaires pour manipuler un dispositif traditionnel.

Selon l'invention, les capteurs permettent ainsi une interprétation des intentions de mouvement du praticien et compenser les contraintes entre les différentes interfaces ce qui procure une meilleure ergonomie au dispositif, i.e. le rendent plus facilement manipulable.

Ainsi, par exemple, le dispositif d'irradiation est configuré pour prendre au moins une configuration d'utilisation, dans laquelle le bras 6-axes est dans une position déployée, et une configuration de rangement et/ou de déplacement dans laquelle le bras 6-axes est alors dans une position repliée. Dans cette configuration, le dispositif d'irradiation présente alors une meilleure compacité, ce qui le rend plus facilement mobile au besoin.

Le dispositif d'irradiation, et plus particulièrement le système d'irradiation comporte la poignée de manipulation.

L'au moins un capteur d'effort est placé entre la poignée de manipulation et le bras 6-axes.

Mais s'il est placé entre le bras 6-axes et le système d'irradiation, les efforts transmis sont très importants car le système d'irraditation, qui est positionné à l'extrémité libre du bras 6-axes, pèse environ 150 kg.

Ainsi, dans un exemple de réalisation particulièrement pratique pour réduire ces efforts, l'au moins un capteur d'effort est placé entre la poignée de manipulation et la source de rayonnement.

Dans un exemple de réalisation privilégié, le dispositif d'irradiation comporte au moins deux, voire trois, capteurs d'effort.

La poignée de manipulation comporte par exemple une anse ou un volant fixe ; elle peut néanmoins comporter toute partie du dispositif pouvant transmettre des efforts à l'au moins un capteur d'effort afin de déplacer et orienter la source de rayonnement.

Le volant peut par exemple entourer une sortie de la source de rayonnement pour ne pas obstruer le rayonnement émis et offrira ainsi une prise pour le praticien quelle que soit sa position.

Dans un exemple de réalisation, le dispositif d'irradiation permet notamment de réaliser des traitements en mode flash.

Un dispositif capable d'opérer en mode flash est détaillé dans le document EP3071292 susmentionné par exemple.

En particulier ici, afin de pouvoir opérer en mode flash, il est nécessaire d'avoir un maximum de puissance de rayonnement. Pour maximiser la puissance disponible, la source hyperfréquence a donc été positionnée au plus proche de la source de rayonnement, ce qui permet de minimiser les pertes de puissance électromagnétique entre la source hyperfréquence et la cavité accélératrice de faisceau de particules, source du rayonnement utile.

Grâce à l'invention, il est ainsi possible de réaliser un dispositif capable de délivrer plus de puissance de rayonnement, pouvant alors opérer en mode flash, mais qui ne soit pas plus encombrant que les systèmes d'art antérieur connus.

En outre, il y avait un préjugé de l'homme du métier à positionner à la fois une source hyperfréquence et une source de rayonnement, type LINAC, en un même ensemble fixé en bout de bras du fait de poids important correspondant.

En effet, dans les dispositifs antérieurs, la source hyperfréquence était généralement utilisée comme contrepoids à la source de rayonnement ; une telle disposition facilitait l'équilibrage du dispositif mais induisait de fortes pertes de puissance électromagnétique hyperfréquence.

Or, positionner à la fois la source hyperfréquence et la source de rayonnement en bout de bras s'est en fait avéré possible grâce aux bras 6-axes désormais disponibles.

En contrepartie, il est cependant préférable de limiter les surpoids pouvant être induits par d'autres composants en bout de bras.

La source hyperfréquence désigne ici une source d'ondes électromagnétiques configurée pour produire un champ électromagnétique de fréquence d'au moins 300 MHz, de préférence dans la bande S des fréquences électromagnétiques (i.e. entre 2 GHz et 4 GHz) ou dans la bande C (i.e. entre 4GHz et 8 GHz) ou encore dans la bande X (i.e. entre 8 GHz et 12 GHz).

Le système d'irradiation est par exemple configuré pour émettre un rayonnement ionisant et délivrer au moins une dose de ce rayonnement ionisant, d'au moins 20 Gy, par exemple 30 Gy, en moins de 100 ms, voire moins de 1 µs.

Selon un exemple de réalisation privilégié, la source de rayonnement comporte un accélérateur linéaire d'électrons dit LINAC.

Ce système d'irradiation est par exemple configuré pour émettre du rayonnement ionisant par impulsions à une fréquence de répétition (f) comprise entre 10 Hz et 1 kHz, chaque impulsion ayant une durée (d) comprise entre 10 ns et 100 µs par exemple.

Par exemple, le système d'irradiation est ici configuré pour délivrer une dose par impulsion comprise entre 1 Gy et 10 Gy.

Dans un exemple de réalisation avantageux, le système d'irradiation, voire par exemple la source de rayonnement, comporte un détecteur ultra-rapide, par exemple un détecteur état solide en carbure de silicium ou en diamant, ou un détecteur à une ou plusieurs chambres d'ionisation, ou encore un transformateur de courant (i.e. de marque Bergoz par exemple) si le rayonnement ionisant est un rayonnement de particules chargées, telles que des électrons ou des protons.

Un détecteur ultra-rapide est de préférence ici positionné à la sortie de la source du faisceau de rayonnement ionisant de façon à être traversé par la totalité du flux de rayonnement de la source de rayonnement.

De cette façon, le détecteur ultra-rapide est configuré pour surveiller la dose de rayonnement délivrée à une cible.

Un détecteur ultra-rapide est ici un détecteur configuré pour détecter une dose de rayonnement ionisant en moins de 0,01 ns et à des débits de dose d'au moins 0,01 Gy/s, voire 25 Gy/s, voire 50 Gy/s, voire de préférence 250 Gy/s, voire encore 500 Gy/s ou même encore 1000 Gy/s.

Un tel détecteur permet ainsi de détecter une dose de rayonnement ionisant qui est produite en moins de 0,01 ns et à des débits de dose d'au moins 0,01 Gy/s, voire 25 Gy/s, voire 50 Gy/s, voire de préférence 250 Gy/s, voire encore 500 Gy/s ou même encore 1000 Gy/s.

Dans un exemple de réalisation intéressant, le système d'irradiation comporte un carter dans lequel sont placées la source hyperfréquence et la source de rayonnement, et sur lequel est fixée la poignée de manipulation ; l'au moins un capteur d'effort étant intercalé entre la poignée de manipulation et une surface du carter.

Selon une option intéressante, la base comporte en outre un système de stabilisation configuré pour compenser un poids induit par le système d'irradiation positionné à l'extrémité libre du bras 6-axes.

Le système de stabilisation permet de mieux compenser un effet de bras de levier ou de porte-à-faux dû au positionnement du système de rayonnement et pour procurer une plus grande portée au bras 6-axes et éviter un basculement du dispositif.

Le système de stabilisation comporte par exemple au moins une béquille. Selon un aspect intéressant, le système de stabilisation comporte en outre une planche escamotable configurée pour prendre une position déployée et une position escamotée, la planche en position déployée étant alors en vis-à-vis du rayonnement.

Une telle planche permet par exemple d'éviter que le rayonnement ne traverse une paroi, par exemple un plancher de la salle où se trouve le dispositif, ou tout autre objet qu'il est préférable de préserver du rayonnement.

La base peut aussi comporter, par exemple, une source de puissance de la source de rayonnement ionisant pour que le dispositif puisse fonctionner en mode flash.

La source de puissance désigne ici une source de haute tension électrique. Selon une autre option, la base peut aussi comporter un système de déplacement omnidirectionnel, comportant par exemple des roues.

Le dispositif d'irradiation peut ainsi être déplacé dans toute direction.

Dans un exemple de réalisation intéressant, les roues de la base sont holonomes. Le système de déplacement permet ainsi le mouvement de rotation du dispositif d'irradiation autour d'un axe quelconque perpendiculaire à la surface du sol sur lequel le dispositif se déplace. En particulier le dispositif peut ainsi tourner sur lui-même. Le système de déplacement de la base, en particulier de telles roues, permet également le déplacement en translation du dispositif d'irradiation au sol dans n'importe quelle direction sans rotation. Les roues holonomes de la base permettent également des mouvements simultanés de rotation et de translation du dispositif d'irradiation. L'avantage d'un tel système de déplacement est la manoeuvrabilité du dispositif d'irradiation pour venir se positionner dans un espace encombré ou exigu. Dans un exemple de réalisation, le dispositif d'irradiation, voire par exemple le système d'irradiation comporte en outre un applicateur configuré pour être fixé en sortie de la source de rayonnement.

L'utilisation d'un bras 6-axes permet à l'applicateur d'être positionné et orienté comme le souhaite le praticien, ce qui permet d'atteindre toute cible par le rayonnement, quelle que soit la position de la cible dans un patient. L'applicateur forme ici une interface entre la cible et la source de rayonnement. Il peut être maintenu par le praticien au regard de la cible tandis que la source de rayonnement portée par le bras à 6-axes est approchée par un autre praticien pour être raccordée à l'applicateur.

L'approche peut être manuelle et assistée par le bras à 6-axes motorisé et commandé par les efforts exercés sur le ou les capteurs d'effort, notamment par la poignée de manipulation.

L'approche finale et l'alignement de l'axe de la source de rayonnement sur l'axe de l'applicateur peuvent être automatisés. Pour cela, le dispositif d'irradiation comporte par exemple un système de raccordement comportant au moins un repère de localisation de l'applicateur et un détecteur, de préférence fixé sur le robot, par exemple sur la source de rayonnement.

Le détecteur permet de localiser l'applicateur par rapport à la source de rayonnement. La localisation de l'applicateur par rapport à la source de rayonnement permet à son tour de commander les mouvements du bras 6-axes porteur de la source de rayonnement de manière à aligner cette dernière avec l'axe de l'applicateur pour l'accoster et le fixer automatiquement sans intervention spécifique d'un opérateur.

Surtout lors, d'un cas non-invasif, et selon l'invention, l'applicateur est fixé à la source de rayonnement, possiblement manuellement et/ou indirectement via le ou les capteurs d'effort.

Ainsi, l'applicateur n'est pas seulement aligné avec la source de rayonnement, sans contact direct, mais est fixé en sortie de la source de rayonnement. Le praticien peut ainsi déplacer, orienter et positionner l'ensemble source de rayonnement et applicateur sur la cible à traiter en manipulant l'applicateur, ou même tout autre élément configuré pour bouger la source de rayonnement et transmettre des efforts au(x) capteur(s) d'effort.

Ainsi, selon l'invention, la poignée de manipulation comporte l'applicateur.

L'au moins un capteur d'effort est alors ici intercalé entre l'applicateur et une surface du carter du système d'irradiation. De ce fait, l'applicateur peut exercer des efforts sur l'au moins un capteur d'effort de sorte à servir de poignée de manipulation.

Lorsque, par exemple, la poignée de manipulation comporte à la fois un applicateur et un volant, le praticien peut excercer un couple important en tenant la poigné d'une main et l'applicateur de l'autre, lui permattant de manipuler facilement le système d'irradation, et en particulier de positionner une extrémité libre de l'applicateur plus facilement et avec une meilleure précision vis-à-vis de la cible.

L'invention, selon un exemple de réalisation, sera bien comprise et ses avantages apparaitront mieux à la lecture de la description détaillée qui suit, donnée à titre indicatif et nullement limitatif, en référence aux dessins annexés dans lesquels :
[Fig. 1] la figure 1 représente schématiquement un dispositif d'irradiation selon un exemple de réalisation de l'invention ;
[Fig. 2] la figure 2 présente un schéma de dispositif d'irradiation flash selon un exemple de réalisation de l'invention ;
[Fig. 3] la figure 3 représente le dispositif de la figure 2 avec le bras en extension ;
[Fig. 4] la figure 4 représente le dispositif de la figure 3 avec le bras replié, pour le déplacer et le ranger ; et
[Fig. 5] la figure 5 montre un exemple de géométrie et d'interfaces de la source de rayonnement ionisant, de capteurs d'effort, de la poignée de manipulation et de l'applicateur.

Les éléments identiques représentés sur les figures précitées sont identifiés par des références numériques identiques.

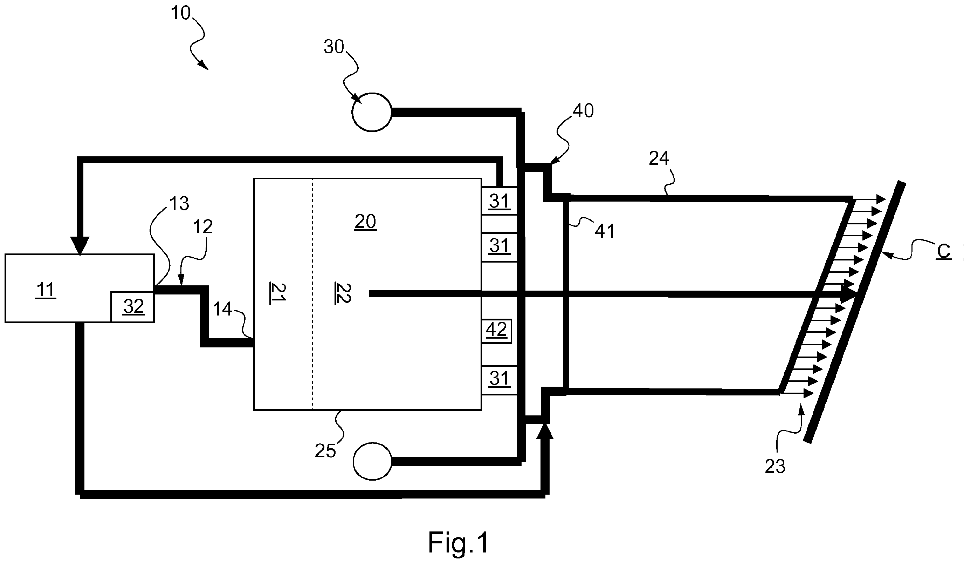

Un dispositif d'irradiation 10 pour irradier une cible C est représenté schématiquement figure 1.

Le dispositif d'irradiation 10 comporte principalement une base 11 et un bras 6-axes 12.

Le bras 6-axes 12 comporte une première extrémité 13 par laquelle il est fixé à la base 11 et une seconde extrémité 14 est dite extrémité libre.

A la première extrémité 13, le bras 6-axes 12 comporte par exemple une interface configurée pour fixer le bras à la base 11.

Ici, à son extrémité libre 14, le bras 6-axes 12 est muni d'un système d'irradiation 20.

Le système d'irradiation 20 est ici rigidement fixé sur l'extrémité libre 14 du bras 6-axes 12.

Le système d'irradiation 20 comporte ici principalement une source hyperfréquence 21 et une source de rayonnement 22 alimentée par la source hyperfréquence 21.

La source de rayonnement 22 est ici configurée pour émettre un faisceau 23, par exemple un faisceau d'électrons.

La source de rayonnement 22 est par exemple un LINAC.

Le système d'irradiation 20, voire la source de rayonnement 22, comporte aussi un détecteur ultra-rapide (non représenté) configuré pour surveiller une quantité de dose de rayonnement délivrée à une cible.

Le détecteur ultra-rapide est de préférence ici positionné en sortie de la source de rayonnement ionisant de façon à être traversé par la totalité du flux de rayonnement.

Un détecteur ultra-rapide est ici un détecteur configuré pour détecter une dose de rayonnement ionisant en moins de 0,01 ns et à des débits de dose d'au moins 0,01 Gy/s, voire 25 Gy/s, voire 50 Gy/s, voire de préférence 250 Gy/s, voire encore 500 Gy/s ou même encore 1000 Gy/s.

Un tel détecteur permet ainsi de détecter une dose de rayonnement ionisant qui est produite en moins de 0,01 ns et à des débits de dose d'au moins 0,01 Gy/s, voire 25 Gy/s, voire 50 Gy/s, voire de préférence 250 Gy/s, voire encore 500 Gy/s ou même encore 1000 Gy/s.

Le détecteur ultra-rapide peut être un détecteur à état solide en carbure de silicium ou en diamant, ou un détecteur à une ou plusieurs chambres d'ionisation, ou encore un transformateur de courant si le rayonnement ionisant est un rayonnement de particules chargées, telles que des électrons ou des protons.

Dans le présent exemple de réalisation, le système d'irradiation 20 comporte un carter 25. La source hyperfréquence 21 et la source de rayonnement 22 sont ici confinées dans le carter 25.

Le dispositif d'irradiation 10 comporte en outre une poignée de manipulation 30, qui est de préférence solidaire de la source de rayonnement 22.

En particulier ici, la poignée de manipulation 30 est fixée sur le carter 25.

La poignée de manipulation 30 comporte par exemple une anse.

Afin de rendre le dispositif d'irradiation collaboratif, le dispositif d'irradiation 10 comporte des capteurs d'effort 31, ici trois capteurs d'effort 31.

Les capteurs d'effort 31 sont ici placés entre la poignée de manipulation 30 et la source de rayonnement 22, et plus précisément ici, les capteurs d'effort 31 sont intercalés entre la poignée de manipulation 30 et une surface du carter 25. Dans un exemple de réalisation préféré, les trois capteurs sont disposés en triangle.

Ainsi, lorsqu'un praticien manipule la poignée de manipulation 30 les capteurs d'effort 31 captent les efforts transmis à la poignée de manipulation 30 et transmettent un signal correspondant à une unité de contrôle-commande 32. En conséquence, l'unité de contrôle-commande 32 commande le bras 6-axes pour collaborer au positionnement et à l'orientation de la source de rayonnement 22 par rapport à la cible C.

L'unité de contrôle-commande 32 est ici représentée dans la base 11.

L'unité de contrôle-commande 32 est donc configurée pour recevoir des informations des capteurs d'effort et commander le bras 6-axes en fonction des informations des capteurs d'effort reçues.

Le système d'irradiation 20 comporte aussi ici un applicateur 24 positionné en sortie de la source de rayonnement 22. Ici, l'applicateur est fixé rigidement à une partie de la poignée 30 au moyen d'un système de fixation 40 configuré pour solidariser l'applicateur en sortie de la source de rayonnement 22. L'applicateur 24 est par exemple un tube, par exemple un tube en plexi-glace. Dans un exemple de réalisation, le système de fixation 40 comporte aussi un système d'accostage 41 et un détecteur de position 42.

Le détecteur de position 42 est par exemple placé sur le système d'irradiation 20 et préférablement sur la source de rayonnement 22.

Dans le cas où l'applicateur 24 est maintenu par un praticien en position vis-à-vis de la cible C sans être encore relié de quelque manière que ce soit en sortie de la source de rayonnement 22, le système de fixation 40 est configuré pour commander le bras 6-axes 12 de manière à fixer la source de rayonnement 22 à l'applicateur 24, dans la position dans laquelle il est maintenu.

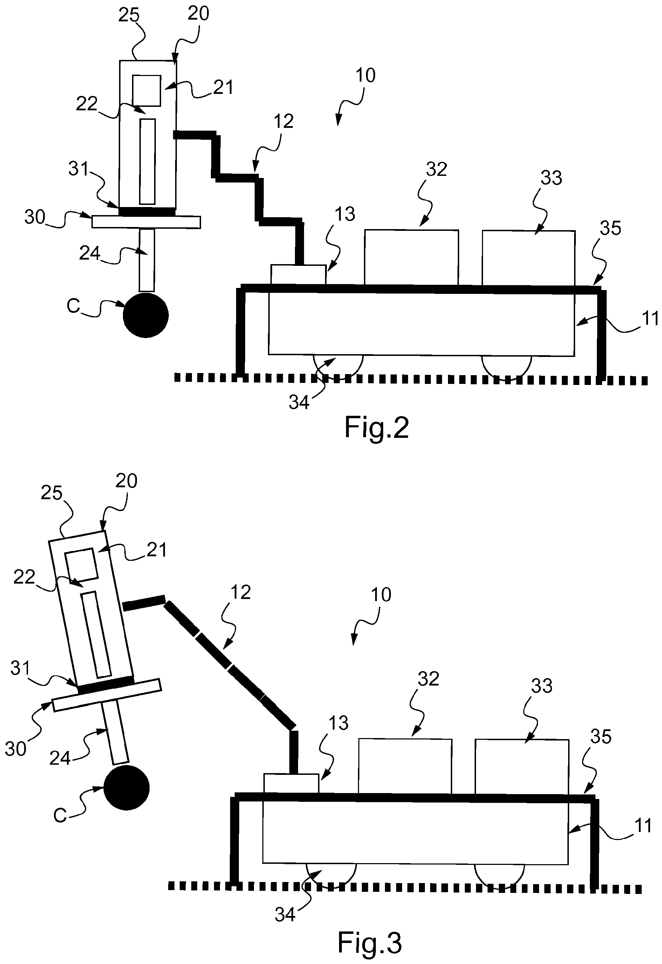

Le détecteur de position 42 détecte la position de l'applicateur 24, transmets des informations correspondantes à l'unité de contrôle-commande 32 qui commande le bras 6-axes 12 pour positionner la source de rayonnement 22 et qui active le système de fixation 40 de manière à fixer l'applicateur 24 dans sa position maintenue par le praticien en sortie de la source de rayonnement 22. La base 11 comporte donc au moins l'unité de contrôle-commande 32. Comme le montre la figure 2, la base 11 peut aussi comporter, par exemple, une source de puissance 33 de la source de rayonnement ionisant pour que le dispositif puisse fonctionner en mode flash.

La base 11 peut aussi comporter un système de déplacement omnidirectionnel 34, comportant par exemple des roues, par exemple holonomes. Le dispositif d'irradiation 10 peut ainsi être déplacé simultanément en translation et en rotation dans toute direction.

Enfin, la base 11 comporte de préférence un système de stabilisation 35.

Le système de stabilisation 35 est configuré pour immobiliser, de manière stable, le dispositif d'irradiation 10.

Le système de stabilisation 35 comporte par exemple des béquilles, voire aussi de préférence une planche escamotable (non représentée) configurée pour prendre une position déployée et une position escamoté, la planche en position déployée étant alors en vis-à-vis du rayonnement.

La présence d'une telle planche permet ainsi d'utiliser le dispositif d'irradiation à différents endroits en limitant les risques que le rayonnement traverse une paroi présente en vis-à-vis du rayonnement.

A titre illustratif, les figures 2 à 4 montre le dispositif d'irradiation 10 selon différentes configurations.

A la figure 2, le dispositif d'irradiation 10 est dans une configuration d'utilisation dans laquelle l'applicateur est positionné au regard de la cible C. Le bras 6-axes 12 est alors dans une position déployée.

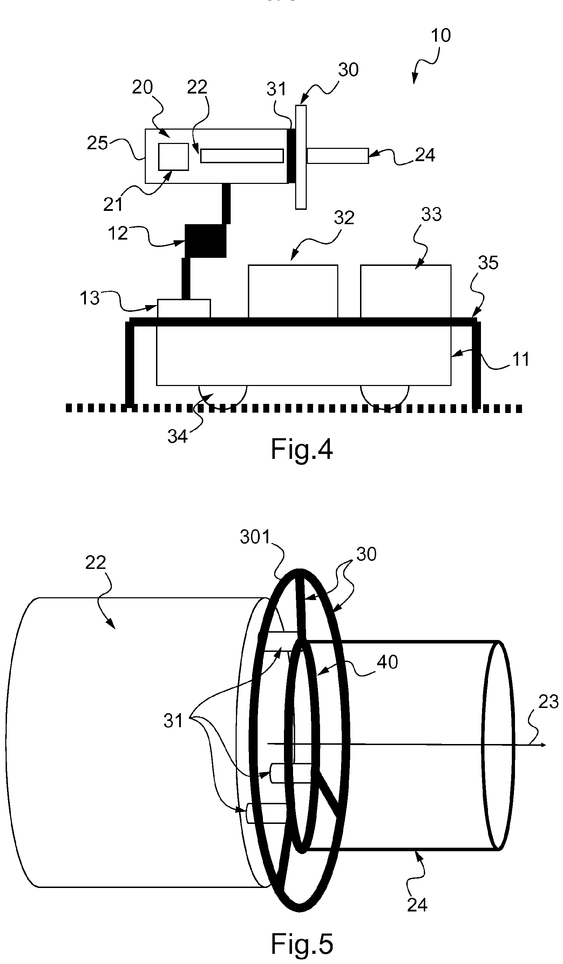

A la figure 3, le dispositif d'irradiation 10 est aussi dans une configuration d'utilisation dans laquelle l'applicateur est positionné au regard de la cible C, mais le bras 6-axes 12 est alors dans une position déployée en extension, c'est-à-dire développé au maximum. En effet, le dispositif d'irradiation selon l'invention permet d'atteindre des zones à traiter plus variées sur une cible C. A la figure 4, le dispositif d'irradiation 10 est dans une configuration de rangement et/ou déplacement. Le bras 6-axes 12 est alors dans une position repliée. Le dispositif d'irradiation présente alors une meilleure compacité, ce qui le rend plus facilement mobile.

La figure 5 illustre plus en détails un exemple de réalisation de la poignée de manipulation 30 et de capteurs d'effort 31.

Dans cet exemple, l'applicateur 24 est rigidement fixé, solidarisé, à une partie de la poignée de manipulation 30, par exemple au moyen du système de fixation 40, rigide, entre l'applicateur 24 et la partie de la poignée de manipulation 30.

Ainsi, l'applicateur fait partie de la poignée de manipulation 30. Un praticien peut alors déplacer et positionner le système d'irradiation (20) en maintenant l'applicateur 24.

La partie de la poignée de manipulation 30 est ici formée par un volant 301.

Le volant 301 peut ainsi entourer une sortie de la source de rayonnement 22 pour ne pas obstruer le rayonnement émis.

Le volant 301 est rigidement relié à la source de rayonnement 22, et à une interface de liaison entre le volant 301 et la source de rayonnement 22, trois capteurs d'effort 31 sont positionnés, en triangle, ici dans un même plan.

Les capteurs d'efforts 31 sont ainsi configurés pour transmettre des signaux correspondant aux efforts appliqués via la poignée à l'unité de contrôle-commande 32, et l'unité de contrôle-commande 32 est configurée pour générer des signaux correspondants pour commander des mouvements du bras 6-axes 12, afin de collaborer au positionnement et à l'orientation de la source de rayonnement 22 par rapport à la cible C à viser.

## Revendications

1. Dispositif d'irradiation (10), configuré pour irradier une cible (C), comportant :
- Un robot 6-axes comportant une base (11) et un bras 6-axes (12), dont une première extrémité (13) est fixée à la base et une seconde extrémité (14) est dite extrémité libre ;
- Un système d'irradiation (20), comportant une source hyperfréquence (21) et une source de rayonnement (22) alimentée par la source hyperfréquence (21), le système d'irradiation (20) étant positionné à l'extrémité libre (14) du bras 6-axes ;
- Une poignée de manipulation (30), solidaire de la source de rayonnement (22), comportant un applicateur (24) configuré pour être fixé en sortie de la source de rayonnement (22) ;
- Au moins un capteur d'effort (31) placé entre la poignée de manipulation (30) et le bras 6-axes (12) ; et
- Une unité de contrôle-commande (32) configurée pour recevoir des informations du capteur d'effort (31) et commander le bras 6-axes (12) en fonction des informations du capteur d'effort (31) reçues.

2. Dispositif selon la revendication 1, dans lequel le dispositif d'irradiation (10) est configuré pour prendre au moins une configuration d'utilisation, dans laquelle le bras 6-axes (12) est dans une position déployée, et une configuration de rangement et/ou déplacement dans laquelle le bras 6-axes (12) est alors dans une position repliée.

3. Dispositif selon l'une quelconque des revendications 1 ou 2, dans lequel la source de rayonnement comporte un LINAC.

4. Dispositif selon l'une quelconque des revendications 1 à 3, dans lequel le système d'irradiation comporte un carter (25) dans lequel sont placées la source hyperfréquence (21) et la source de rayonnement (22), et sur lequel est fixée la poignée de manipulation (30) ; le capteur d'effort (31) étant intercalé entre la poignée de manipulation (30) et une surface du carter (25).

5. Dispositif selon l'une quelconque des revendications 1 à 4, dans lequel le système d'irradiation comporte au moins deux capteurs d'effort (31).

6. Dispositif selon l'une quelconque des revendications 1 à 5, dans lequel l'au moins un capteur d'effort (31) est placé entre la poignée de manipulation (30) et la source de rayonnement (22).

7. Dispositif selon l'une quelconque des revendications 1 à 6, dans lequel la base comporte en outre un système de stabilisation (35) configuré pour compenser un poids induit par le système d'irradiation positionné à l'extrémité libre du bras 6-axes.

8. Dispositif selon la revendication 7, 2. dans lequel le système de stabilisation (35) comporte une planche escamotable configurée pour prendre une position déployée et une position escamotée, la planche en position déployée étant alors en vis-à-vis du rayonnement.

9. Dispositif selon l'une quelconque des revendications 1 à 8, dans lequel le système d'irradiation (20) est configuré pour émettre un rayonnement ionisant et délivrer une dose de rayonnement ionisant d'au moins 20 Gy en moins de 100 ms.

10. Dispositif selon l'une quelconque des revendications 1 à 9, dans lequel poignée de manipulation (30) comporte un volant (301) entourant une sortie de la source de rayonnement (22).

11. Dispositif selon l'une quelconque des revendications 1 à 10, dans lequel base (11) comporte une source de puissance (33) de la source de rayonnement (22), ladite source de puissance (33) étant une source de haute tension électrique.

12. Dispositif selon l'une quelconque des revendications 1 à 11, dans lequel base (11) comporte un système de déplacement omnidirectionnel (34).

13. Dispositif selon l'une quelconque des revendications 1 à 12, dans lequel le système d'irradiation (20) comporte un détecteur ultra-rapide configuré pour surveiller la dose de rayonnement délivrée à la cible (C).

14. Dispositif selon la revendication 13, dans lequel le détecteur ultra-rapide est configuré pour détecter une dose de rayonnement en moins de 0,01 ns et à des débits de dose d'au moins 0,01 Gy/s.

## Patentansprüche

1. Bestrahlungsvorrichtung (10), die konfiguriert ist, um ein Ziel (C) zu bestrahlen, umfassend:
- einen 6-Achsen-Roboter, der eine Basis (11) und einen 6-Achsen-Arm (12) umfasst, von dem ein erstes Ende (13) an der Basis befestigt ist und ein zweites Ende (14) als freies Ende bezeichnet wird;
- ein Bestrahlungssystem (20), das eine Mikrowellenquelle (21) und eine Strahlungsquelle (22) umfasst, die von der Mikrowellenquelle (21) gespeist wird, wobei das Bestrahlungssystem (20) am freien Ende (14) des 6-Achsen-Arms positioniert ist;
- einen fest mit der Strahlungsquelle (22) verbundenen Bediengriff (30), der einen Applikator (24) umfasst, der konfiguriert ist, um am Auslass der Strahlungsquelle (22) befestigt zu werden;
- mindestens einen Kraftsensor (31), der zwischen dem Bediengriff (30) und dem 6-Achsen-Arm (12) angeordnet ist; und
- eine Steuereinheit (32), die konfiguriert ist, um Informationen vom Kraftsensor (31) zu empfangen und den 6-Achsen-Arm (12) in Abhängigkeit von den empfangenen Informationen des Kraftsensors (31) anzusteuern.

2. Vorrichtung nach Anspruch 1, wobei die Bestrahlungsvorrichtung (10) konfiguriert ist, um mindestens eine Gebrauchskonfiguration, in der sich der 6-Achsen-Arm (12) in einer ausgefahrenen Stellung befindet, und eine Verstau- und/oder Bewegungskonfiguration, in der sich der 6-Achsen-Arm (12) dann in einer eingefahrenen Stellung befindet, einzunehmen.

3. Vorrichtung nach einem der Ansprüche 1 oder 2, wobei die Strahlungsquelle einen LINAC umfasst.

4. Vorrichtung nach einem der Ansprüche 1 bis 3, wobei das Bestrahlungssystem ein Gehäuse (25) umfasst, in dem die Mikrowellenquelle (21) und die Strahlungsquelle (22) angeordnet sind und an dem der Bediengriff (30) befestigt ist; wobei der Kraftsensor (31) zwischen dem Bediengriff (30) und einer Oberfläche des Gehäuses (25) eingefügt ist.

5. Vorrichtung nach einem der Ansprüche 1 bis 4, wobei das Bestrahlungssystem mindestens zwei Kraftsensoren (31) umfasst.

6. Vorrichtung nach einem der Ansprüche 1 bis 5, wobei der mindestens eine Kraftsensor (31) zwischen dem Bediengriff (30) und der Strahlungsquelle (22) angeordnet ist.

7. Vorrichtung nach einem der Ansprüche 1 bis 6, wobei die Basis weiter ein Stabilisierungssystem (35) umfasst, das konfiguriert ist, um ein Gewicht, das durch das am freien Ende des 6-Achsen-Arms positionierte Bestrahlungssystem verursacht wird, zu kompensieren.

8. Vorrichtung nach Anspruch 7, wobei das Stabilisierungssystem (35) ein versenkbares Brett umfasst, das konfiguriert ist, um eine ausgefahrene Stellung und eine versenkte Stellung einzunehmen, wobei das Brett in ausgefahrener Stellung dann der Strahlung gegenüberliegt.

9. Vorrichtung nach einem der Ansprüche 1 bis 8, wobei das Bestrahlungssystem (20) konfiguriert ist, um eine ionisierende Strahlung zu emittieren und eine Dosis ionisierender Strahlung von mindestens 20 Gy in weniger als 100 ms abzugeben.

10. Vorrichtung nach einem der Ansprüche 1 bis 9, wobei der Bediengriff (30) ein Handrad (301) umfasst, das einen Auslass der Strahlungsquelle (22) umgibt.

11. Vorrichtung nach einem der Ansprüche 1 bis 10, wobei die Basis (11) eine Leistungsquelle (33) der Strahlungsquelle (22) umfasst, wobei die Leistungsquelle (33) eine elektrische Hochspannungsquelle ist.

12. Vorrichtung nach einem der Ansprüche 1 bis 11, wobei die Basis (11) ein omnidirektionales Bewegungssystem (34) umfasst.

13. Vorrichtung nach einem der Ansprüche 1 bis 12, wobei das Bestrahlungssystem (20) einen ultraschnellen Detektor umfasst, der konfiguriert ist, um die an das Ziel (C) abgegebene Strahlungsdosis zu überwachen.

14. Vorrichtung nach Anspruch 13, wobei der ultraschnelle Detektor konfiguriert ist, um eine Strahlungsdosis in weniger als 0,01 ns und mit Dosisleistungen von mindestens 0,01 Gy/s zu erfassen.

## Claims

1. Irradiating device (10) configured for irradiating a target (C), comprising:
- A 6-axis robot comprising a base (11) and a 6-axis arm (12), of which a first end (13) is attached to the base and a second end (14) is designated free end;
- An irradiating system (20), comprising a microwave frequency source (21) and a radiation source (22) supplied by the microwave frequency source (21), the irradiating system (20) being positioned at the free end (14) of the 6-axis arm;
- A manipulating handle (30), joined to the radiation source (22), comprising an applicator (24) configured to be fastened at an exit of the radiation source (22);
- At least one load sensor (31) placed between the manipulating handle (30) and the 6-axis arm (12); and
- A control-actuation unit (32) configured to receive information from the load sensor (31) and control the 6-axis arm (12) according to the information received from the load sensor (31).

2. Device according to claim 1, in which the irradiating device (10) is configured to adopt at least a use configuration, in which the 6-axis arm (12) is in an extended position, and a configuration for storage and/or movement in which the 6-axis arm (12) is then in a folded position.

3. Device according to any one of claims 1 or 2, in which the radiation source comprises a LINAC.

4. Device according to any one of claims 1 to 3, in which the irradiating system comprises a casing (25) in which are placed the microwave frequency source (21) and the radiation source (22), and on which is fastened the manipulating handle (30) the load sensor (31) being interposed between the manipulating handle (30) and a surface of the casing.

5. Device according to any one of claims 1 to 4, in which the irradiating system comprises at least two load sensors (31).

6. Device according to any one of claims 1 to 5, in which the at least one load sensor (31) is placed between the manipulating handle (30) and the radiation source (22).

7. Device according to any one of claims 1 to 6, in which the base further comprises a stabilizing system (35) configured to compensate for a weight induced by the irradiating system positioned at the free end of the 6-axis arm.

8. Device according to claim 7, in which the stabilizing system (35) comprises a retractable board configured to take an extended position and a retracted position, the board in extended position then facing opposite the radiation.

9. Device according to any one of claims 1 to 8, in which the irradiating system (20) is configured to emit ionizing radiation and deliver a dose of ionizing radiation of at least 20 Gy in less than 100 ms.

10. Device according to any one of claims 1 to 9 in which the manipulating handle (30) comprises a wheel (301) surrounding an exit of the radiation source (22).

11. Device according to any one of claims 1 to 10, in which the base (11) comprises a power source (33) of the radiation source (22), said power source (33) being a high voltage electrical source.

12. Device according to any one of claims 1 to 11, in which the base (11) comprises an omnidirectional movement system (34).

13. Device according to any one of claims 1 to 12, in which the irradiating system (20) comprises an ultra-fast sensor configured to monitor the radiation dose delivered to the target (C).

14. Device according to claim 13, in which the ultra-fast sensor is configured to detect a dose of radiation in less than 0.01 ns and at dose throughputs of at least 0.01 Gy/s.
